# EUROPEAN PATENT SPECIFICATION

(11) **EP 0 633 798 B1**
(45) Date of publication and mention of the grant of the patent: **07.05.2003**
(21) Application number: 93908616.1
(22) Date of filing: 29.03.1993
(51) Int. Cl.: A61M 29/02, A61F 2/04, A61B 5/00

(54) **VASCULAR FILTER**
BLUTGEFÄSSFILTER
FILTRE VASCULAIRE

(30) Priority: 31.03.1992 US 861253; 08.07.1992 US 910631
(43) Date of publication of application: 18.01.1995
(73) Proprietor: BOSTON SCIENTIFIC CORPORATION, Natick, MA 01760-1537 (US)
(72) Inventor: HEATH, Kevin, R., Providence, RI 02908 (US); ABELE, John, E., Concord, MA 01742 (US)
(74) Representative: Deans, Michael John Percy
(86) International application number: PCT/US93/02871
(87) International publication number: WO 93/019803

(56) References cited:
- EP-A- 0 121 447
- EP-A- 0 433 011
- WO-A-90/01300
- WO-A-91/04716
- WO-A-92/11815
- WO-A-92/19310
- WO-A-94/16646
- DE-U- 9 206 170
- FR-A- 2 479 685
- US-A- 4 425 908
- US-A- 4 793 348
- US-A- 4 950 227
- US-A- 5 025 799
- US-A- 5 069 226

## Description

This invention relates to metal medical components to be used inside the body, and specifically to vascular filters including medical wire.

Noninvasive medical procedures reduce the risk of surgery by introducing medical devices to a body cavity through small incisions or body orifices. The devices are carefully designed so that they may be controlled from the proximal end remaining outside the body to carry out the required treatment at the desired location inside the body. In one of the most common noninvasive techniques, angiography, a device, such as a guidewire, balloon angioplasty catheter or the like, is advanced and torqued at its proximal end to steer the device through a blood vessel to the position of an occlusion at which point a medical procedure such as balloon angioplasty and/or positioning of an endoprosthesis is carried out.

Typically, X-ray fluoroscopy is used to view the medical device within the body cavity to monitor placement and operation. The device may also be viewed by X-ray film after placement. To use these techniques, particularly with small devices which may be difficult to view, the medical device must include some radiopaque material, more dense than the surrounding tissue, to provide sufficient contrast on an X-ray image. A highly dense, and therefore particularly radiopaque, metal is usually incorporated with the portion of the medical device used inside the body for this purpose.

WO-A-9104716 discloses a anti-pulmonary embolism filter of the type made of an elastic wire with remanent spring effect. The filter is configured like a spiral with three non-jointed turns, the medial turn having a diameter which is larger than the diameter of the two other turns and selected to be approximately equal to the value of the semi-circumference of the vena cava in the area where the filter has to be implanted so that the filter is held in place by flattening the vena cava. In a preferred embodiment, the distal end of the wire of the filter is provided with a small radio-opaque cylinder, which is rounded off at its free end, to facilitate positioning of the filter in the vena cava.

In interventional medicine, wires can be used for a variety of purposes such as tracking, stenting, filtering, conducting (electric current, ultrasound energy, etc.) and marking. Desirable attributes of these wires vary with application, but include properties such as stiffness, tensile strength, elasticity, radiopacity, weldability, flexural life, conductivity, etc. These properties are hard to find in single-material constructions. It is possible to achieve optimum properties by creating a multiple material coaxial construction. In medical wires, for example, it can be very desirable to have high radiopacity along with elasticity and strength. This may be accomplished by combining a radiopaque material with an elastic material. Although it is possible to put either material on the inside or outside, it would be preferable to put the dense radiopaque material (e.g., tantalum) on the inside (core) since dense materials are generally less elastic and the elastic material (e.g., titanium or nickel-titanium alloy) on the outside (clad). The clad or "skin" of the wire will undergo more deformation in bending than the core, so the elastic component is best positioned at the skin.

The invention relates to a vascular filter including a wire-form member to be used within the body with properties that can be tailored to a particular application. The wire-form member is formed of preferably two or more dissimilar metals joined together to form a unitary member. Typically, each metal contributes a desirable property to the filter which is not substantially impaired by the presence of the other metal. In particularly preferred filters, one metal provides enhanced radiopacity. In these embodiments, the filter includes a wire-form member which comprises a metal outer member having a predetermined density and an exposed outer surface and a core including a metal having a density greater than the outer member to enhance radiopacity. The core is secured within and substantially enclosed by the outer member. Preferably, the wire-form member is in the form of a wire configured such that the mechanical properties, for example, the elastic properties, of the metal forming the outer member are affected by the core to a desired degree so that the wire has a desired overall performance suitable for its intended use. Preferably, the mechanical properties of the outer longitudinal member dominate the properties of the wire yet the radiopacity is substantially enhanced by the denser core. The invention also allows increased radiopacity of a metal filter without adversely affecting and in some cases improving other important properties such as the biocompatibility, size or other performance characteristics. These performance advantages can be realized by proper selection of the material of the outer member and core, their relative size, and geometrical configuration. The performance characteristics of the wire-form member may be dictated by the filter into which the radio-opaque wire-form member is to be incorporated.

The term "metal" as used herein includes electropositive chemical elements characterized by ductility, malleability, luster, and conductivity of heat and electricity, which can replace the hydrogen of an acid and forms bases with the hydroxyl radical and including mixtures including these elements and alloys. Many examples are given below.

The invention provides a vascular filter for placement in a blood vessel to filter clots in blood flowing through the blood vessel within the body, the filter including a wire-form member comprising an extended metal outer member and a core within said outer member comprising a metal different than the metal of said outer member, said core being secured within and enclosed by said out member, characterised in that said outer member comprises a metal of predetermined density and said core comprises a metal having a density greater than said outer member to enhance radiopacity of said filter.

Various preferred embodiments may include one or more of the following features. The extended metal outer member is comprised of a metal of predetermined density and the core is comprised of a metal having a density substantially greater than the outer member to enhance radiopacity of the filter. The wire -form member is in the form of a medical wire, wherein the metal outer member is a longitudinal member and the radiopaque core is positioned along the axis of the longitudinal member. The radiopaque core has a density of about 9.9 g/cc or greater. The core is selected from the group consisting of tungsten, tantalum, rhenium, iridium, silver, gold, bismuth, platinum and alloys thereof. The core has a modulus of elasticity of about 550 GPa or less. The core has a modulus of elasticity of about 200 GPa or less. The outer member is selected from the group consisting of superelastic alloys, precursor alloys of superelastic alloys, stainless steel, and titanium and its alloys.

The superelastical alloy is nitinol. The core is about 1 to 40% of the cross-sectional dimension of the wire -form member. The core is about 25% or more of the cross-sectional dimension of the wire-form member. The core is about 28% or less of the cross-sectional dimension of the wire-form member. The cross-sectional dimension of the wire-form member is less than about 0.635mm (0.025 inch). The outer member has a cross-section of about 0.114 to 0.203mm (0.0045 to 0.008 inch) and the core member has a cross-section of about 0.036 to 0.050mm (0.0014 to 0.00195 inch) inner diameter. The core is a solid metallic member. The outer member has portions of varying dimension. The outer member has a taper portion. The core has a constant inner dimension in portions corresponding to the varying outer dimension of the outer member. The wire-form member is in the form of elastic leg members of a vascular filter. The outer member and core are of circular cross-sectional configuration.

In various preferred embodiments, the core material is tantalum, the outer material is nitinol, and the cross sectional dimension of the wire-form member is about 0.635mm (0.025 inch) or less. The outer member has a cross-section of about 0.114 to 0.203mm (0.0045 to 0.008 inch) and the core member has a cross-section of about 0.036 to 0.050mm (0.0014 to 0.00195 inch) inner diameter. The outer member has portions of varying dimension such as a taper portion. The core has a constant inner dimension in portions corresponding to the varying outer dimension of the outer member. The cross-sectional dimension of the portion is about 0.889 to 0.940mm (0.035 to 0.037 inch). The core is about 0.127mm (0.005 inch) in diameter. The component is in the form of elastic leg members of a vascular filter.

In one preferred embodiment, the wire-form member is adapted to be subjected to elastic deformation to enable the wire-form member to be forced into a characteristic deformed configuration during a stage of use and to elastically self-recover from the deformation when deformation forces are relieved and the core of the wire-form member has a modulus of elasticity of 550 Gpa or less.

In various preferred embodiments, the wire-form member comprises a draw-form. The outer material is nitionol. The core material is tantalum.

We first briefly describe the drawings.
Fig. 1 is a longitudinal cross-sectional view of a medical wire for use in a vascular filter according to the invention; while Fig. 1a is a cross-sectional view taken along the lines aa in Fig. 1;
Fig. 2 is a schematic illustration of a wire for use in a vascular filter according to the invention in a stressed, bent configuration;
Fig. 3 is a graph of load versus displacement for several wires for use in a vascular filter according to the invention;
Fig. 4 is a schematic of a blood clot filtration device of the invention for implantation in a blood vessel.

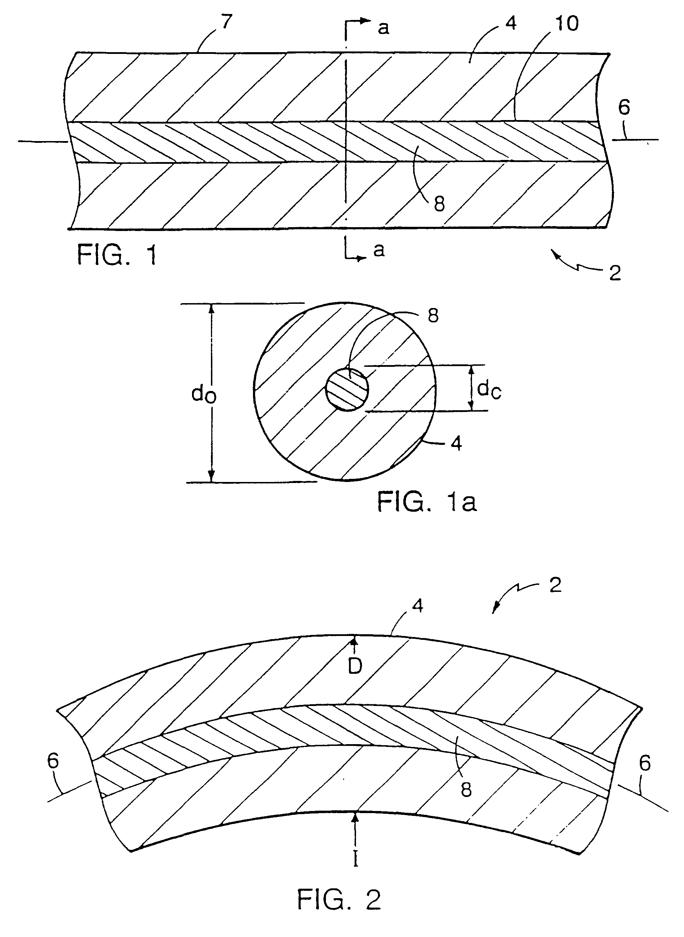

Referring to Figs. 1 and 1a, a preferred medical wire for use in a vascular filter of the invention is a medical wire 2 that includes a longitudinal outer member 4 with an axis 6. The longitudinal member 4 is formed of a metal having desirable properties, such as high elasticity and biocompatibility of its exposed outer surface 7. (The surface 7 may include a non-metal coating of, e.g., fluorocarbons, silicones, hydrophilic and lubricous biocompatible materials.) About the axis 6 is a core material 8 including a metal with a density greater than the longitudinal member 4 to enhance the radiopacity of the wire. The core 8 is bonded to and substantially enclosed by the outer member 4 such that it does not have any substantial exposed surface and therefore does not contact body tissue when positioned within the body during use.

As illustrated, preferably the core 8 is a continuous solid member in intimate contact and bonded with the outer member 4 without the formation of substantial voids in the interface 10 between the core and outer member. Preferably, the elastic properties of the wire 2 are dominated by the elastic properties of the longitudinal member 4. The core material 8 enhances the radiopacity of the wire 2 but preferably does not substantially effect the mechanical performance of the wire. In preferred embodiments, the cross-sectional dimension of the core (d_{c}) is less than about 70% (but typically greater than about 1% or 10%) of the outer cross-sectional dimension (dₒ) of the wire, more preferably between about 40% and 25%. The wire is especially useful in applications where the filter must be sized small, such as for use in the vascular system. The wire is particularly useful for enhancing radiopacity of devices with dimensions of about 0.635mm (0.025 inch) or less.

Referring to Fig. 2, the wire 2 is shown in a bent position, as it may be, when in use in the filter of the present invention positioned within the body. The inner and outer portions (i) and (o), experience a wide range of tension and compression as the wire is bent. An advantage of the invention, is that by positioning the core material 8 near the axis 6, the range of tension and compression imposed on the core is reduced and a wide latitude of dense, substantially radiopaque materials can be used which otherwise might not be suitable for their response to bending or other mechanical properties.

The relative dimension of the core and outer member and the particular materials used for these elements are selected based on the desired over-all mechanical properties of the wire and the degree to which x-ray visibility is to be enhanced, since the core affects the mechanical properties of the wire compared to a solid wire formed of the outer material, and the radiopacity is a function of the sum of the mass between an x-ray beam source and detector. For example, large filters or filters with overlapping portions, may require less radiopaque material to provide sufficient visibility. Similarly, the location of use in the body may affect the amount of dense material needed for sufficient visibility. The visibility of a filter can be tested by known techniques such as ASTM Designation F640-79 "Standard Test Method for Radiopacity of Plastics for Medical Use". In this test, the background densities which may be encountered clinically are mimicked by an aluminum plate positioned over the wire having various thicknesses.

The properties of the outer member metal and core which may be considered include density, modulus of elasticity (in annealed and hardened states), biocompatability (primarily a factor for the material of the outer longitudinal member), flexural stiffness, durability, tensile and compression strength, acoustic impedance (as discussed in a further embodiment below) and the required radiopacity and resolution.

Preferably, for elastic members, the outer member is formed of a continuous solid mass of a highly elastic biocompatible metal such as a superelastic or pseudoelastic metal alloy, for example, a nitinol (e.g., 55% nickel, 45% titanium). Other examples of superelastic materials include, e.g., Silver-Cadmium (Ag-Cd), Gold-Cadmium (Au-Cd), Gold-Copper-Zinc (Au-Cu-Zn), Copper-Aluminum-Nickel (Cu-Al-Ni), Copper-Gold-Zinc (Cu-Au-Zn), Copper-Zinc (Cu-Zn), Copper-Zinc-aluminum (Cu-Zn-Al), Copper-Zinc-Tin (Cu-Zn-Sn), Copper-Zinc-Xenon (Cu-Zn-Xe), Iron Beryllium (Fe₃Be), Iron Platinum (Fe₃Pt), Indium-Thallium (In-Tl), iron-manganese (Fe-Mn) Nickel-Titanium-Vanadium (Ni-Ti-V), Iron-Nickel-Titanium-Cobalt (Fe-Ni-Ti-Co) and Copper-Tin (Cu-Sn). See Schetsky, L. McDonald, *"Shape Memory Alloys",* Encyclopedia of Chemical Technology (3rd ed.), John Wiley & Sons, 1982, vol. 20. pp. 726-736 for a full discussion of superelastic alloys. Other examples of metals suitable for the outer member include stainless steel, titanium and various alloys of these metals and the precursor of superelastic alloys. Precursors of superelastic alloys are those alloys which have the same chemical constituents as superelastic alloys, but have not been processed to impart the superelastic property. Such alloys are further described in co-owned and co-pending WO-A-91151152.

The core material is preferably a continuous solid mass, but may also be in a powder-form. The core includes a metal that is relatively dense to enhance radiopacity. Preferably, the core metal has a density of about 9.9 g/cc or greater. Most preferably, the core is formed of tantalum (density = 16.6 g/cc). Other preferred materials and their density include tungsten (19.3 g/cc), rhenium (21.2 g/cc), bismuth (9.9 g/cc), silver (16.49 g/cc), gold (19.3 g/cc), platinum (21.45 g/cc), and iridium (22.4 g/cc). In some cases barium can be used in the core. The core may be formed of alloys such as those including the above materials. Typically, the core is somewhat stiffer than the outer membrane. Preferably, the core metal has a low modulus of elasticity, e.g., preferably below about 550 GPa, e.g., such as tantalum (186 GPa). A smaller difference between the modulus of elasticity between the outer material and core, results in a smaller variation of the modulus from that of the outer material in the wire for use in the filter of the invention. For larger differences, a smaller core may be used so as to produce a wire in which the elastic properties are dominated by the outer material.

The outer member and core may be in many cross-sectional geometric configurations, such as circular, square, triangular, hexagonal, octagonal, trapezoidal and the geometrical configuration of the core may differ from that of the longitudinal member. For example, the wire may be rectangular in cross-section with a rectangular core or triangular or hexagonal in cross-section with a circular core. The wire may also take on the form of tubing with a lumen within the core extending along the axis of the wire. The wire may also include successive layers of different metals to form a composite system. The core may extend intermittently along the axis in a desired pattern.

The wire may be formed, for example, by drilling a relatively large rod of the outer member material to form a lumen, positioning a rod of core material in the lumen, sealing the ends of the lumen, e.g., by crimping and drawing as known in the art, through a series of dies of decreasing diameter until the desired outer diameter is achieved. The wire may be heat treated to anneal, harden or impart superelastic properties. Other methods of formation may be, e.g., by coating the core with the desired outer material such as by electro- or electroless plating. The materials used in the outer member and core are also selected based on their workability for forming the wire, including factors such as machinability, for forming the longitudinal member into a tubular piece and the core member into a rod shaped piece, stability in gaseous environments at annealing temperatures, properties related to welding, drawing, forging, swaging, the ability to accept coatings such as adhesives, polymers, lubricants and practical aspects such as cost and availability.

Other examples follow.

### Example 1

A 500 foot length of wire (0.132mm (0.0052 inch) in diameter) having an outer member formed of a precursor of a nitinol (55% Ni/45% Ti) superelastic alloy and a core material of tantalum (0.044mm (0.00175 inch) in diameter) is formed by drilling a 6.35mm (0.25 inch) diameter bore in a 19.05mm (0.75 inch) rod of the outer member material and providing in the drilled lumen a tantalum member of substantially matched outer diameter. The rod is mechanically forged in a standard hot forging and rolling apparatus, then hammered such that no substantial voids between the core and outer longitudinal member are present. One end of the rod is sealed and the opposite end is cold drawn longitudinally through a dye to the final diameter. Initially, the outer member of the wire is the precursor of a superelastic alloy, i.e., it has not been heat treated to impart the superelastic property.

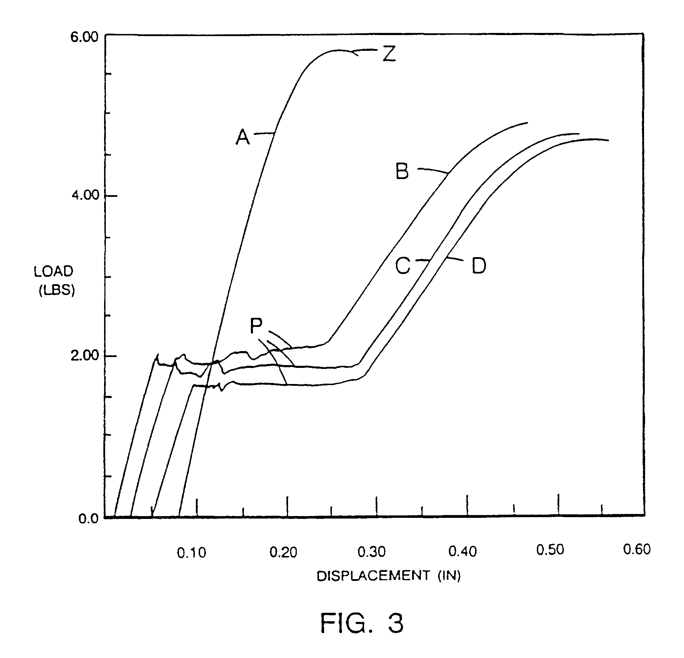

Referring to Fig. 3, load versus displacement curves are illustrated. (For clarity, curves c, D and A are offset, successively, 0.635mm (0.025 inch) on the x-axis Curve A illustrates the wire as discussed in the above paragraph prior to heat annealing which induces the superelastic property; the wire exhibits substantially linear elastic strain as a function of stress to a break point z. Curves B, C, D illustrate stress/strain curves after annealing the wire at 460°C for 3 minutes, 5 minutes and 15 minutes. respectively. As these curves illustrate, the superelastic nature of the wire is substantially preserved, as evidenced by the substantial plateaus (p) on the stress/strain curve, despite the presence of the tantalum core. Also as illustrated, the stress at which constant displacement occurs decreases with increasing annealing, as would be expected with a superelastic material. The mechanical properties of the wire, therefore, are dominated by the nitinol alloy, despite the presence of the tantalum core.

Referring to Table I, the modulus of elasticity and plateau stress calculated based on stress-strain measurements as above, are compared for the wires of the invention and a solid wire of Ni-Ti alloy.

As the results in Table I illustrate, the modulus of elasticity of the wires for use in the filter of the invention was varied less than 30% compared to the solid Ni-Ti wire. The plateau stress of the wires for use in the filter of the invention using a superelastic outer member was varied less than about 10% compared to a solid Ni-Ti superelastic wire. The wire formed as described exhibits about 30% or more enhanced x-ray visibility over a wire of the same thickness formed of solid material. Preferably, wires as described, dominated by the mechanical properties of the outer superelastic member and exhibiting generally satisfactory radiopacity have outer diameter (dₒ) of about 0.203 to 0.114mm (0.008 to 0.0045 inch) with a core diameter (d_{c}) of about 0.036 to 0.050mm (0.0014 to 0.00195 inch).

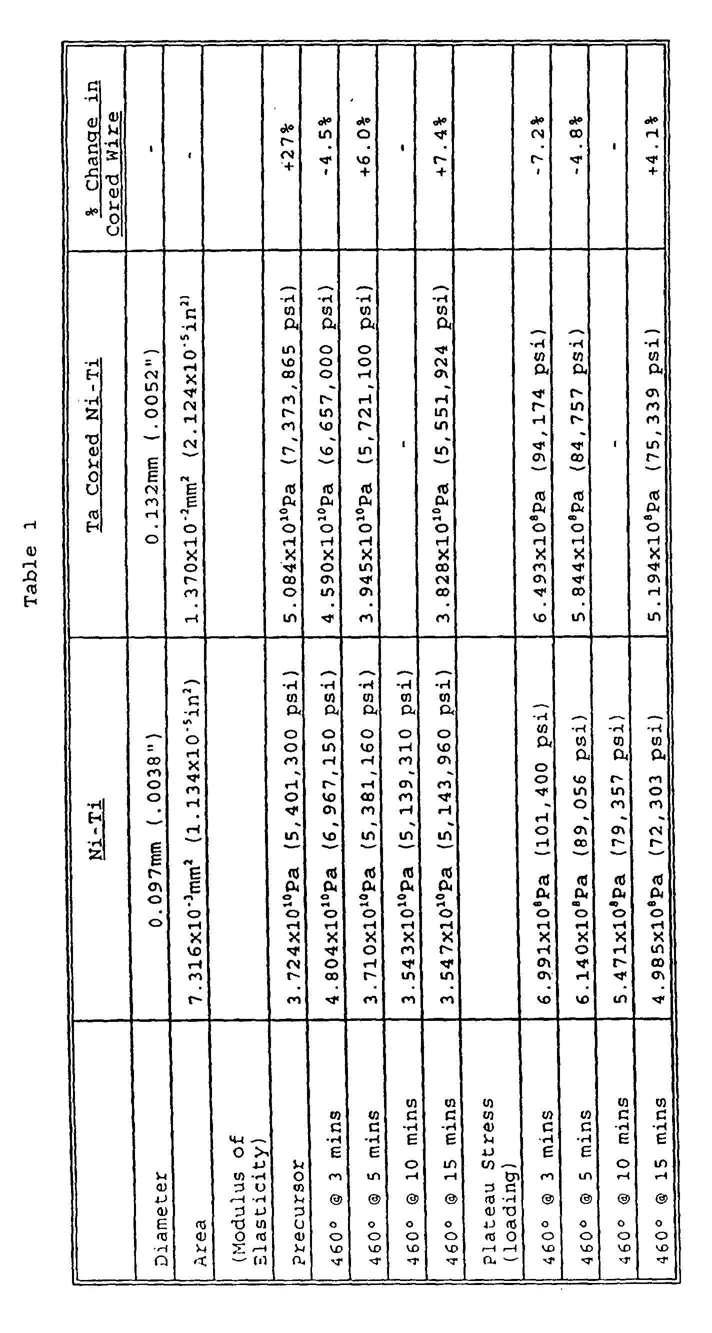

### Example 2

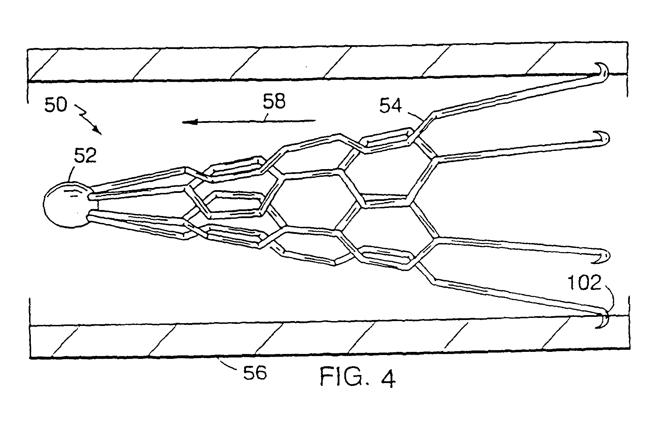

Referring to Fig. 4, a wire be used in a blood clot filtration device according to the invention of the type positioned within a blood vessel. The filtration device 50 includes a nose cone 52 downstream relative to blood flow 58 and a series of legs 54. The legs include hook members 102 which are embedded in the wall 56 of the body lumen to secure the filter therein. A filter of this type is further described in Herms U.S. 4,817,600 and E1-Nou Nou U.S. 5,059,205. The wires 54 are formed as described herein including a radiopaque core within an elastic longitudinal member. Preferably, the overall diameter of the wire is 0.889 to 0.940mm (0.035 to 0.037 inch) with a core diameter of about 0.127mm (0.005 inch). The outer member is preferably nitinol, stainless steel or titanium and the core is tantalum. It will be understood that smaller diameter wires for smaller filtration device legs may also be used, in which case, a dense core is particularly useful for enhancing radiopacity.

## Claims

1. A vascular filter (50) for placement in a blood vessel to filter clots in blood flowing through the blood vessel within the body, the filter including a wire-form member comprising an extended metal outer member (4) and a core (8) within said outer member (4) comprising a metal different than the metal of said outer member, said core (8) being secured within and enclosed by said outer member (4), **characterised in that** said outer member (4) comprises a metal of predetermined density and said core (8) comprises a metal having a density greater than said outer member (4) to enhance radiopacity of said device.

2. A vascular filter according to claim 1 wherein said metal outer member (4) is a longitudinal member and said core (8) is positioned along the axis (6) of said longitudinal member.

3. A vascular filter according to claim 1 or claim 2 wherein said core (8) has a density of 9.9 g/cc or greater.

4. A vascular filter according to any one of the preceding claims wherein said core (8) is selected from the group consisting of tungsten, tantalum, rhenium, iridium, silver, gold, bismuth, platinum and alloys thereof.

5. A vascular filter according to any one of the preceding claims wherein said outer member (4) is selected from the group consisting of superelastic alloys, precursor alloys of superelastic alloys, stainless steel, and titanium and its alloys.

6. A vascular filter according to claim 5 wherein the outer member (4) comprises nitinol.

7. A vascular filter according to any one of the preceding claims wherein the core (8) comprises tantalum.

8. A vascular filter device according to any one of the preceding claims wherein the core (8) comprises from 1 to 40% of the cross-sectional dimension of the wire-form member.

9. A vascular filter according to any one of claims 1 to 7 wherein the core (8) comprises from 10% to 50% of the cross-sectional dimension of the wire-form member.

10. A vascular filter according to any one of the preceding claims wherein the core (8) is 25% or more of said cross-sectional dimension of the wire-form member.

11. A vascular filter according to claim 10 wherein the core (8) is 28% or less of the cross-sectional dimension of the wire-form member.

12. A vascular filter according to any one of the preceding claims wherein the cross-sectional dimension of the wire-form member is less than 0.635mm (0.025 inch).

13. A vascular filter according to claim 12 wherein the outer member (4) has a cross-section of 0.114 to 0.203mm (0.0045 to 0.008 inch) and the core (8) has a cross-section of 0.036 to 0.050mm (0.0014 to 0.00195 inch) inner diameter.

14. A vascular filter according to any one of the preceding claims wherein said core (8) is a solid metallic member.

15. A vascular filter according to any one of the preceding claims wherein said outer member (4) and core (8) are of circular cross-sectional configuration.

16. A vascular filter according to any one of the preceding claims wherein the core (8) is in intimate contact and bonded with the outer member (4).

17. A vascular filter according to any one of the preceding claims wherein the wire-form member comprises a plurality of elastic leg members (54) .

18. A vascular filter according to claim 17 wherein the or each leg member (54) is provided with a hook member (102) to secure the filter within the blood vessel.

19. A vascular filter according to any one of the preceding claims wherein the filter includes a nose cone (52).

20. A vascular filter according to any one of the preceding claims wherein said wire-form member is adapted to be subjected to elastic deformation to enable said wire-form member to be forced into a characteristic deformed configuration during a stage of use and to elastically self-recover from said deformation when deformation forces are relieved and the core (8) of said wire-form member has a modulus of elasticity of 550 Gpa or less.

21. A vascular filter according to claim 20 wherein said core (8) has a modulus of elasticity of 200 Gpa or less.

22. A vascular filter according to any one of the preceding claims wherein said outer member (4) comprises a lower density metal having more elasticity than the metal of the core (8).

## Patentansprüche

1. Gefäßfilter (50) zum Anordnen in einem Blutgefäß, um Gerinsel im Blut, das innerhalb des Körpers durch das Blutgefäß strömt, zu filtern, wobei das Filter ein Drahtformbauteil einschließt, das ein verlängertes Metallaußenbauteil (4) und einen Kern (8) innerhalb des Außenbauteils (4) umfasst, das ein anderes Metall umfasst als das Metall des Außenbauteils, wobei der Kern (8) innerhalb des Außenbauteils (4) befestigt ist und von diesem umringt ist, **dadurch gekennzeichnet, dass** das Außenbauteil (4) ein Metall vorherbestimmter Dichte umfasst und der Kern (8) ein Metall mit einer Dichte umfasst, die größer ist als die des Außenbauteils (4), um die Radiopazität der Vorrichtung zu erhöhen.

2. Gefäßfilter gemäß Anspruch 1, wobei das Metallaußenbauteil (4) ein Längsbauteil ist und der Kern (8) entlang der Achse (6) des Längsbauteils angebracht ist.

3. Gefäßfilter gemäß Anspruch 1 oder Anspruch 2, wobei der Kern (8) eine Dichte von 9,9 g/cm³ oder größer aufweist.

4. Gefäßfilter gemäß einem der vorangegangenen Ansprüche, wobei der Kern (8) ausgewählt ist aus Wolfram, Tantal, Rhenium, Iridium, Silber, Gold, Bismut, Platin und Legierungen davon.

5. Gefäßfilter gemäß einem der vorangegangenen Ansprüche, wobei das Außenbauteil (4) ausgewählt ist aus superelastischen Legierungen, Vorstufenlegierungen von superelastischen Legierungen, rostfreiem Stahl und Titan und seinen Legierungen.

6. Gefäßfilter gemäß Anspruch 5, wobei das Außenbauteil (4) Nitinol umfasst.

7. Gefäßfilter gemäß einem der vorangegangenen Ansprüche, wobei der Kern (8) Tantal umfasst.

8. Gefäßfilter gemäß einem der vorangegangenen Ansprüche, wobei der Kern (8) 1 bis 40% der Querschnittsabmessung des Drahtformbauteils umfasst.

9. Gefäßfilter gemäß einem der Ansprüche 1 bis 7, wobei der Kern (8) 10 bis 50% der Querschnittsabmessung des Drahtformbauteils umfasst.

10. Gefäßfilter gemäß einem der vorangegangenen Ansprüche, wobei der Kern (8) 25% oder mehr der Querschnittsabmessung des Drahtformbauteils umfasst.

11. Gefäßfilter gemäß Anspruch 10, wobei der Kern (8) 28% oder weniger der Querschnittsabmessung des Drahtformbauteils umfasst.

12. Gefäßfilter gemäß einem der vorangegangenen Ansprüche, wobei die Querschnittsabmessung des Drahtformbauteils weniger als 0,635 mm (0,025 Zoll) beträgt.

13. Gefäßfitter gemäß Anspruch 12, wobei das Außenbauteil (4) einen Querschnitt von 0,114 bis 0,203 mm (0,0045 bis 0,008 Zoll) aufweist und der Kern (8) einen Querschnitt von 0,036 bis 0,050 mm (0,0014 bis 0,00195 Zoll) Innendurchmesser aufweist

14. Gefäßfilter gemäß einem der vorangegangenen Ansprüche, wobei der Kern (8) ein Massivmetallbauteil ist.

15. Gefäßfilter gemäß einem der vorangegangenen Ansprüche, wobei das Außenbauteil (4) und der Kem (8) von Rundquerschnittsgestalt sind.

16. Gefäßfilter gemäß einem der vorangegangenen Ansprüche, wobei sich der Kern (8) in innigem Kontakt befindet mit und verbunden ist mit dem Außenbauteil (4).

17. Gefäßfilter gemäß einem der vorangegangenen Ansprüche, wobei das Drahtformbauteil eine Mehrzahl von elastischen Beinbauteilen (54) umfasst.

18. Gefäßfilter gemäß Anspruch 17, wobei das oder jedes Beinbauteil (54) mit einem Hakenbauteil (102) bereitgestellt wird, um das Filter innerhalb des Blutgefäßes zu befestigen.

19. Gefäßfilter gemäß einem der vorangegangenen Ansprüche, wobei das Filter einen Nasenkegel (52) umfasst.

20. Gefäßfilter gemäß einem der vorangegangenen Ansprüche, wobei das Drahtformbauteil daran angepasst ist, elastischer Verformung unterzogen zu werden, um zu ermöglichen, dass das Drahtformbauteil während der Phase der Verwendung in eine charakteristische verformte Konfiguration gezwungen wird, und es sich aus der Verformung elastisch selbsttätig wiederherstellt, wenn die Verformungskräfte abnehmen, und der Kern (8) des Drahtformbauteils einen Elastizitätsmodul von 550 Gpa oder weniger aufweist.

21. Gefäßfilter gemäß Anspruch 20, wobei der Kern (8) einen Elastizitätsmodul von 200 Gpa oder weniger aufweist.

22. Gefäßfilter gemäß einem der vorangegangenen Ansprüche, wobei das Außenbauteil (4) ein Metall mit einer geringeren Dichte mit mehr Elastizität als das Metall des Kerns (8) umfasst.

## Revendications

1. Filtre vasculaire (50) destiné à être placé dans un vaisseau sanguin pour filtrer les caillots du sang qui circule dans le vaisseau sanguin à l'intérieur du corps, le filtre comprenant un organe en forme de fil métallique comprenant un organe externe (4) de métal déployé et une âme (8) placée dans l'organe externe (4) et comprenant un métal différent du métal de l'organe externe, l'âme (8) étant fixée dans l'organe externe (4) et entourée par celui-ci, **caractérisé en ce que** l'organe externe (4) est formé d'un métal d'une masse volumique prédéterminée et l'âme (8) est formée d'un métal de masse volumique supérieure à celle de l'organe externe (4) afin que l'opacité radiographique du dispositif soit accrue.

2. Filtre vasculaire selon la revendication 1, dans lequel l'organe externe métallique (4) est un organe longitudinal et l'âme (8) est disposée le long de l'axe (6) de l'organ longitudinal.

3. Filtre vasculaire selon la revendication 1 ou 2, dans lequel l'âme (8) a une masse volumique de 9,9 g/cm³ ou plus.

4. Filtre vasculaire selon l'une quelconque des revendications précédentes, dans lequel l'âme (8) est sélectionnée dans le groupe constitué par le tungstène, le tantale, le rhénium, l'iridium, l'argent, l'or, le bismuth, le platine, et leurs alliages.

5. Filtre vasculaire selon l'une quelconque des revendications précédentes, dans lequel l'organe externe (4) est sélectionné dans le groupe formé par les alliages superélastiques, les alliages précurseurs d'alliage superélastique, l'acier inoxydable, et le titane et ses alliages.

6. Filtre vasculaire selon la revendication 5, dans lequel l'organe externe (4) est formé de "Nitinol".

7. Filtre vasculaire selon l'une quelconque des revendications précédentes, dans lequel l'âme (8) est formée de tantale.

8. Filtre vasculaire selon l'une quelconque des revendications précédentes, dans lequel l'âme (8) forme 1 à 40 % de la dimension de la section de l'organe en forme de fil.

9. Filtre vasculaire selon l'une quelconque des revendications 1 à 7, dans lequel l'âme (8) forme 10 à 50 % de la dimension de la section de l'organe en forme de fil.

10. Filtre vasculaire selon l'une quelconque des revendications précédentes, dans lequel l'âme (8) forme au moins 25 % de la dimension de la section de l'organe en forme de fil.

11. Filtre vasculaire selon la revendication 10, dans lequel l'âme (8) constitue au maximum 28 % de la dimension de la section de l'organe en forme de fil.

12. Filtre vasculaire selon l'une quelconque des revendications précédentes, dans lequel la dimension de la section de l'organe en forme de fil est inférieure à 0,635 mm (0,025 pouce).

13. Filtre vasculaire selon la revendication 12, dans lequel l'organe externe (4) a une section comprise entre 0,114 et 0,203 mm (0,0045 et 0,008 pouce) et l'âme (8) a une section de diamètre interne compris entre 0,036 et 0,050 mm (0,0014 et 0,00195 pouce).

14. Filtre vasculaire selon l'une quelconque des revendications précédentes, dans lequel l'âme (8) est un organe métallique plein.

15. Filtre vasculaire selon l'une quelconque des revendications précédentes, dans lequel l'organe externe (4) et l'âme (8) ont une configuration circulaire en coupe.

16. Filtre vasculaire selon l'une quelconque des revendications précédentes, dans lequel l'âme (8) est en contact intime avec l'organe externe (4) et est liée à celui-ci.

17. Filtre vasculaire selon l'une quelconque des revendications précédentes, dans lequel l'organe en forme de fil comporte plusieurs organes de branche élastique (54).

18. Filtre vasculaire selon la revendication 17, dans lequel l'organe ou chaque organe de branche (54) a un organe en forme de crochet (102) destiné à fixer le filtre dans le vaisseau sanguin.

19. Filtre vasculaire selon l'une quelconque des revendications précédentes, dans lequel le filtre à un cône de nez (52).

20. Filtre vasculaire selon l'une quelconque des revendications précédentes, dans lequel l'organe en forme de fil est destiné à être soumis à une déformation élastique destinée à permettre à l'organe en forme de fil d'être mis à force à une configuration déformée caractéristique pendant une étape d'utilisation et de se rétablir automatiquement et élastiquement de sa déformation lorsque les forces de déformation sont supprimées, et l'âme (8) de l'organe en forme de fil a un module d'élasticité inférieur ou égal à 550 GPa.

21. Filtre vasculaire selon la revendication 20, dans lequel l'âme (8) a un module d'élasticité inférieur ou égal à 200 GPa.

22. Filtre vasculaire selon l'une quelconque des revendications précédentes, dans lequel l'organe externe (4) est un métal de masse volumique relativement faible ayant une élasticité supérieure à celle du métal de l'âme (8).
